# EUROPEAN PATENT SPECIFICATION

(11) **EP 1 629 847 B1**
(45) Date of publication and mention of the grant of the patent: **13.01.2010**
(21) Application number: 04019119.9
(22) Date of filing: 12.08.2004
(51) Int. Cl.: A61K 36/00

(54) **Herbal AIDS treatment**
Heilkräuterzusammensetzung zur Behandlung von AIDS
Composition à base de plantes pour le traitement du SIDA

(43) Date of publication of application: 01.03.2006
(73) Proprietor: Shengxun, Tian, Kabulonga, Lusaka (ZM)
(72) Inventor: Shengxun, Tian, Kabulonga, Lusaka (ZM); Shengzhi, Tian, Zhengzhou, Henan (CN)
(74) Representative: Maucher, Wolfgang

(56) References cited:
- WO-A-02/32444
- US-A- 031 559
- US-A1- 2002 076 446
- US-A1- 2002 122 833
- US-A1- 2003 091 658
- US-B1- 6 524 627

## Description

### Summary of the invention

The invention relates to certain pharmaceutical (combination) products for use in the treatment of a retroviral infection, in particular HIV infection, especially AIDS, as described in detail below.

### Background of the invention

In some cases, plants, plant parts or plant extracts known from traditional Chinese herbal medicine have been reported to be of use in stimulating the immune system or in the treatment of viral diseases. However, until now no successful medication on herbal basis has become available on the market that makes it possible to treat (at least considerably mitigate) HIV infection, especially, AIDS or other retroviral diseases.

AIDS is estimated to have infected presently around 48 Million people world wide according to recent information from the United Nations. Untreated, AIDS is usually fatal, except for very few cases where spontaneous reduction and even spontaneous cure from the disease was reported.

Several treatments based e.g. on Protease Inhibitors (PI) and Reverse Transcriptase Inhibitors (RT) are known. Usually, two or more such drugs are combined, e.g. in HAART (Highly Active Anti-Retroviral Therapy). Typical treatment schedules involve two nucleoside type RT inhibitors and one PI or one non-nucleoside type RT inhibitor. There are severe adverse side effects, such as a bone marrow suppression, neuropathy, myopathy, lipodystrophia (leading e.g. to loss of fat in the face and increase of fat tissue at certain places, e.g. breast, intra-abdominally and in the neck and shoulder area. Further negative effects are on the liver, leading up to steatohepatitis and liver failure. Various other deleterious side effects are known.

Further, treatment is required for the rest of the life of the patients to be treated, thus leading to long-term toxic effects.

In addition, there is a high risk that, due to mutation and recombination, the causative agent for AIDS, the retrovirus HIV-1 or HIV-2, develops resistance against the drugs used. Further, usually life-long treatment is required.

The treatments up to now involved treatment after the virus has already entered the cells of the immune system that are affected by AIDS, so that the treatment up to now was limited to a situation where the virus already is inside the cells.

Presently, there is a focus on experimental compounds that stop HIV already from entering the cells, rather than combating them only when already in the cells. Switzerland's Roche and US biotech group Trimeris last year launched a first drug of this type, known as fusion inhibitor. However, the corresponding drug (Fuzeon) is expensive, has to be injected twice daily and its sales have been disappointing so far. In the beginning of the year 2004, other drug companies, such as Schering-Plough and Pfizer, have reported on different kinds of entry inhibitors that block a cellular doorway called CCR5 and which can be given orally as a pill. Also Smithkline Beecham is working on such a CCR5 product.

Treatment by inhibition of cell fusion of infected cells and thus inhibition of infection of further cells by humic acids, such as HS-1500, has been reported (see EP 0 537 430) - however, in vivo treatment was not proven to be sufficiently successful.

There are market analyst predictions that the HIV/AIDS market will double in size to US-$ 12 billion by 2012, despite the decision by major companies - under pressure from activists - to slash the cost of medicine in poor countries, e.g. African countries where the epidemic is highly prevalent.

In view of the development of resistance, the different possibilities of interference with the viral infection and the cost of the planned and available drugs, there is an everlasting need for further medications and treatments, e.g. with known or preferably new and/or combined mechanisms of action, that allow for the treatment (at least palliative) of AIDS and other retroviral infections, such as those caused by HTLV-I in humans.

Chinese herbal medicine is known and has reputation as providing products of use in many diseases and disorders, especially aiming at a balanced, integrated approach that is harmonic and not merely centred around treatment of one symptom, in addition showing no or merely few undesired adverse reaction.

US 2003/091658 describes a compulsory combination of four core ingredients (Herba hedyotis diffusa, Rhizoma Polygoni bistortae, Rhizoma Polygoni cuspidate and Schisandra chinensis) plus 6 further ingredients, among them Panax notoginseng, and 4 additional ingredients, among the Angelica sinensis and Astragalus mebranaceous. US 5,178,865 cited in US 2003/091658 names 10 plant extracts, among them from Salvia miltiorrhiza and Isatis tinctoria.

WO 02/32444 also mentions four necessary core components to which inter alia Chinese Angelica, Radix Notoginseng and Radix Astragali may be added "to provide further nutrition".

US 2002/076446, US 2002/031559, US 2002/122833 and US 6,524,627 mention further compositions of possible use in treatment of also virus related diseases.

It is a problem to be solved by the present invention to provide a new pharmaceutical product or formulation that allows for the treatment of retroviral infections such as AIDS, showing advantageous properties, e.g. allowing treatment both for extended periods of time as well as for shorter periods of time, making use of ingredients that are not too toxic and/or providing a further approach compared to the known primarily single-drug or combined chemical entity based treatment of retroviral infections such as AIDS used so far. It is a further problem to provide novel medications and treatments, e.g. with known or preferably new and/or combined mechanisms of action, for the treatment of retroviral infections, such as AIDS.

### General description of the invention

Surprisingly, using a cocktail of extracts and powdered plant materials from plants known at least to a large extent in traditional Chinese herbal medicine can be shown to at least alleviate the symptoms of AIDS and/or reduce the viral load in patients to a considerable extent, thus leading to significant improvement or near cure in the affected patients and allowing a new type of treatment (including prophylaxis).

Without wishing to be bound to this possible mechanism of action, it can also be shown that these cocktails are capable to diminish or even block the fusion of cells that usually happens in HIV infected cells and leads to syncytia with a plurality of cell nuclei. Therefore, at least part of the effect of the new products and formulations against retroviral infection may be due to this mechanism of action.

### Detailed description of the invention

(A) In one embodiment, the invention relates to a pharmaceutical product containing
   (i) either one single dosage form,
      or
   (ii) two dosage forms jointly active against the disease to be treated as kit of parts for simultaneous, and/or sequential use, where each dosage form comprises 4 to 8 of the following components, so that each of
   the single dosage form (i) alone,
   and the two dosage forms as kit of parts (ii) when taken together,
   contains all of the following components in the following relative part per weight amounts:
   an extract of Isatis root 10 - 60,
   an extract of Glycyrrhizae radix 20 - 80,
   an extract of Trichosanthis Kirilowii fruit 5 - 45,
   an extract of Rhizoma pinelliae 0.5 -10,
   an extract of Zingiberis Siccatum rhizome 1 - 6,
   an extract of Astragali radix 10 - 80,
   an extract of Salvia miltiorrhizae radix 3 - 35,
   an extract of Glycyrrhizae baked with honey 2 - 40,
   Trichosanthis Kirilowii radix in powder form 5 - 45,
   Panax quinquefolia in powder form 3 - 50,
   Angelica sinensis in powder form 20 - 80 and
   Cordyceps sinensis in powder form 0.05 - 5;
   and optionally one or more pharmaceutically acceptable carrier and/or coating materials, with or without further additives in accordance with accepted practices of pharmaceutical formulating,
   for use in the treatment of a retroviral infection.

   This embodiment relates to the corresponding product wherein the one type of dosage form (i) , or in the case that two dosage forms are present the two or more dosage forms of the product (ii) when taken together, contain all of the components mentioned in the preceding paragraph, in the absence or presence of one or more pharmaceutically acceptable carrier and/or coating materials, with or without further additives in accordance with accepted practices of pharmaceutical formulating.
(B) More preferably, the product according to the two preceding paragraphs as kit of parts is a product with two dosage forms for sequential or preferably simultaneous administration.
(C) Still more preferably, the invention relates to a product according to any one of the three preceding paragraphs wherein the dosage form is a capsule or preferably a tablet.
(D) Again more preferred, the invention relates to a product according to any one of the preceding four paragraphs wherein the extract components are prepared from a concentrated solution with a specific gravity (related to that of water in all cases where mentioned) of 1.15 to 1.35, preferably 1.2 to 1.3, before the final drying, especially wherein the extract components are obtainable (this preferably meaning obtained where used in the present specification), especially obtained, by aqueous extraction.
(E) Most preferably, the invention relates to a product according to any one of the preceding five paragraphs wherein the components are present in the following relative parts per weight amounts:
   extract of Isatis root 20 to 50, more preferably 30 to 40, most preferably 36;
   extract of Glycyrrhizae radix 30 to 70, preferably 45 to 55, most preferably 51;
   extract of Trichosanthis Kirilowii (especially T. Kirilowii Maxim) fruit 15 to 35, preferably 20 to 30, most preferably 24;
   extract of Rhizoma pinelliae 1 to 6, preferably 1.5 to 5, most preferably 3;
   extract of Zingiberis Siccatum rhizoma in dried form, preferably 1.5 to 5, most preferably 3;
   extract of Astragali radix 20 to 70, preferably 35 to 55, most preferably 45;
   extract of Salvia miltiorrhizae radix 5 to 30, preferably 10 to 25, most preferably 18;
   extract of Glycyrrhizae baked with honey 5 to 30, preferably 10 to 20, most preferably 15;
   Trichosanthis Kirilowii (especially T. K. Maxim) radix in powder form 15 to 35, preferably 20 to 30, most preferably 24;
   Panax quinquefolia in powder form 5 to 35, preferably 10 to 30, most preferably 20
   Angelica sinensis (preferably (Olive.) Diels). in powder form 30 to 70, preferably 40 to 60, most preferably 48; and
   Cordyceps sinensis (preferably (Berk.)) in powder form 0.1 to 1, preferably 0.25 to 0.75, most preferably 0.5.

Other preferred embodiments of the invention are represented in the claims 7 to 12 which are incorporated here by reference.

The invention allows for the use of the following components:
an extract of Isatis root,
an extract of Glycyrrhizae radix,
an extract of Trichosanthis Kirilowii (especially T. K. Maxim) fruit,
an extract of Rhizoma pinelliae
an extract of Zingiberis Siccatum rhizoma,
an extract of Astragali radix,
an extract of Salvia miltiorrhizae radix,
an extract of Glycyrrhizae baked with honey,
Trichosanthis Kirilowii (especially T. K. Maxim) radix in powder form,
Panax quinquefolia in powder form,
Angelica sinensis (preferably Olive.) Diels.) in powder form and
Cordyceps sinensis (preferably (Berk.)) in powder form
in the preparation of a product according to any one of paragraphs (A) to (E) given above.

A process for the manufacture of a pharmaceutical product according to the invention is comprising
(a) admixing one or more components in extract form enumerated in the claims just mentioned with one or more components in powder form in one or more batches;and
(b) adding a pharmaceutically acceptable carrier material to each batch.

The invention also allows for a method of treatment (including prophylaxis) of a patient, that is, a warmblooded animal, especially a human, with a retroviral infection, especially HIV infection, most especially AIDS, comprising administering to said patient in need of such treatment an amount that is effective in the treatment of said disease of a product or a formulation, especially as defined above, according to the invention.

Most preferably, the invention relates to the products given in the Examples.

The general terms or symbols used hereinbefore and hereinafter preferably have within, the context of this disclosure, the following meanings, unless otherwise indicated:

A product with two or more dosage forms for the simultanous, sequential or separate use (use = administration) relates to a kit of parts that comprises one or more dosage forms, e.g. in one box or otherwise characterised as being for combined use. The dosage forms are so chosen that they are separately or especially jointly active against the disease to be treated, e.g. a retroviral infection, such as HIV infection, especially AIDS.

Simultaneous means that the components are administered at approximately the same time, preferably within less than a minute of time difference, especially directly at the same time, for example after a meal.

Sequential means chronically staggered which means that the dosage forms may be given separately in such time intervals that they preferably, in the individual, e.g. human, to be treated, still show a (preferably synergistic) interaction, that is, are jointly therapeutically effective. Whether this is the case, can inter alia be determined by following the blood levels of RANTES which is a cytokine that is anti-retrovirally active and can be shown to display elevated levels in the case of treatment with a formulation or product according to the invention, showing that the relevant active principles of the components are present in the blood of the individual, e.g. human, to be treated at least during certain time intervals.

Separate preferably means that the dosage forms components are administered such that no overlap of measurable blood levels of active principles from the components takes place, that is, they are not in an overlapping manner (at the same time).

The borders are, of course, not rigid.

It is also possible and included as embodiment of the invention that any combination of two or more of simultaneous, sequential or separate use is possible. Any combination preferably means that the dosage forms that are part of the product may be administered at one time point simultaneously, followed by administration of only one dosage form at a later time point and subsequently another dosage form or the combination of two or more dosage forms at a still later time point, and the like.

Alternatively, the components may be present in fixed combination (as mixture) in one single dosage form.

By the term dosage forms (= unit dosage forms), there aremeant pharmaceutical formulations.

Possible pharmaceutical formulations are, for example, those for parenteral or preferably enteral administration.

The components and combinations mentioned hereinbefore and hereinafter are especially useful especially in pharmaceutically acceptable oral or, in a broader aspect of the invention, topical formulations (e.g. creams or lotions or the like that are administered in the vagina or other places where a risk of infection exists e.g. during sexual intercourse).

The pharmaceutical formulations in the case of the kit of parts comprise each 4 to 8 of the components mentioned above and below per unit dosage form, or where all are present in one dosage form all of them, preferably in association with a pharmaceutically acceptable carrier material.

Any one or more conventional carrier materials suitable can be used. Suitable carriers for the preferred oral administration include but are not limited to gelatine, gum arabic, lactose, starch, magnesium stearate, talc, vegetable oils, polyalkylene-glycols and petroleum jelly.

Additionally, additives such as flavouring agents, preservatives, complexing agents, pigments, dyes, stabilizers, tensides, emulsifying agents, wetting agents, solubilizers, buffers and the like may be added in accordance with accepted practices of pharmaceutical formulating.

The pharmaceutical preparations can be made up in any conventional form including inter alia: (a) a solid form for oral administration such as tablets, capsules (e.g. hard or soft gelatine capsules), pills, sachets, powders, granules, and the like; (b) preparations for topical administrations such as solutions, suspensions, ointment, creams, hydrogels, lipogels or micronized powders. The pharmaceutical preparations may be sterilized and/or may contain adjuvants such as preservatives, stabilizers, wetting agents, emulsifiers, salts for varying the osmotic pressure and/or buffers.

For topical administration to the skin or mucous membranes one or more of the aforementioned components are preferably prepared as ointments, tinctures, creams, gels, solution, lotions; and dry powder for inhalation; suspensions, shampoos, hair soaps and perfumes. In fact, any conventional composition can be utilised in this invention.

Wherever used, the term treatment or therapy also includes prophylactic treatment.

Examples for the preferred oral dosage form comprise tablets (including pills), sachets, or capsules of hard or soft gelatine, methylcellulose or of another suitable material easily dissolved in the digestive tract. Each tablet, pill, sachet or capsule can preferably contain from about 10 to about 2000 mg, more preferably from about 20 to about 1500 mg, of one or more of the components mentioned above. The oral dosages contemplated in accordance with the present invention will vary in accordance with the needs of the individual patient (e.g. the condition of the patient, the size, the age, possible interferences with other therapeutic measures and the like) as determined by the prescribing physician.

Generally, however, in the treatment of an adult human, a daily dosage of 4 to 40 g, relating to the weight amount of all components of a product or formulation according to the invention as a total, whether distributed over more than one (preferably two) dosage forms or as combination in one dosage form, more preferably of 20 to 35 g per day, preferably administered at two to six, more preferably 2 to 4, such as 3, different time points, especially after meals, of the patient is utilised. This dosage may be administered according to any dosage schedule determined by the physician in accordance with the requirements of the patient. In the case of children, usually a lower dose is recommended, e.g. in the range of half the dose used for an adult person. In the case of administration of white, red and yellow tablets given in the Examples, preferably 2 to 5, especially 3 yellow, 2 to 5, especially 3 red, and 2 to 5, especially 3 white tablets may be administered three times a day, e.g. after the meals. Treatment preferably is for a period of e.g. at least 3 to 12 months, more preferably more than 6 months.

The dosage for treatment typically depends on the route of administration, the age, weight and disease condition of the individual.

Preferably, the dosage forms according to the invention are capsules or most preferably tablets. Tablets may be covered by coatings customary in pharmaceutical formulating practice, e.g. sugar-comprising lacquers that may be coloured in order to identify different dosage forms.

The preferred relative amount of the one or more (active) components of the dosage forms is such that the dosage form comprises 20 to 100 %, more preferably 50 to 98 %, e.g. 70 to 95 % of the components, the rest being, for example, pharmaceutically one of more components selected from, for example, pharmaceutically acceptable carrier materials and coatings.

Especially in case of opportunistic infections accompanying AIDS, e.g. pneumonia, the products or formulation of the invention can, if need arises, be accompanied by antibiotic and/or in case of opportunistic viral infection antiviral treatment with known drugs, e.g. antibiotics or antiviral drugs, as well as in case of tuberculosis and malaria.

The (active) components used in the realisation of the products and formulations of the present invention are given above. The mentioned components practically all have a long tradition in Chinese herbal medicine.

At least ten of these components are used or present in a product according to the invention (for example where both extract and powder form are used, only one of them may be present), more preferably all of them.

Isatis root comes from the isatis plant that originated in China and India and is now cultivated in the West as well.

Glycyrrhiza (licorice) radix is the root of the perennial shrub Licorice native to the Mediterranean regions, Russia, Middle East and southeast Asia. The roots of three of the varieties (G. glabra, G. inflata and here especially G. uralensis) are officially recognised by Chinese pharmacopoeia.

Trichosanthis Kirilowii (preferably T. K. Maxim) Maxim is used in the present invention both in the form of an extract of the fruit (Chinese Gualou) and as powder of the roots.

Pinellia (ban xia) is a small, stem-like plant native to southern China and Japan. The medically useful part of the plant is its root, or tuber. In the present invention, this is used in the form of an extract.

Zingiber (Ginger) is also a well-known drug or drug component in Chinese medicine. It is used as dried Ginger (Zingiberis Siccatum rhizoma).

Astragalus (especially A. membranaceus), a member of the pea family, is sometimes referred to as "the senior of all herbs" in Chinese medicine and is used in the form of the roots (radix) (Chinese Huang Qi).

Salvia miltiorrhizae radix is also well-known in Chinese medicine, e.g. to remove blood stasis and relieve pain.

Glycyrrhiza baked with honey (Licorice root honey coated or Zhi Gan Cao) is also a well-known active principle or ingredient, e.g. used as extract, in Chinese medicine.

Panax quinquefolia (American Ginseng; Chinese Ren Shen) is here preferably used as the root in powder form.

Angelica sinensis (here especially A. s. (Olive.) Diels.) (in Chinese Deng gui), namely as root, is used here in powder form.

Cordyceps sinensis (here especially C. s. (Berk.)) (aweto; in Chinese: Dong Chong Xia Cao) is from Cordyceps, a rare herb, and has been an important ingredient in Chinese medicine for thousands of years. in powder form

Where "relative parts per weight amounts" are mentioned above and below, this refers to the relative weight amounts of the components (active components) mentioned above or below forming part of the product or formulation according to the invention.

Where "extract" is used in this disclosure, the extracts may be obtained with customary solvents, such as alcohols, e.g. ethanol, or ethers, such as diethyl ether, by extraction with liquid or superfluid gases, such as carbon dioxide, or preferably by extraction with aqueous solutions (e.g. with buffers or salts), more preferably with water. The extraction takes place for each component that is used in extract form alone or in combination with one or more of the other plants or plant parts to be extracted. Preferably, the extraction takes place by heating the solvent, especially water, e.g. by boiling, preferably by heating with water vapor. The extracts are then usually at least partially concentrated, preferably evaporated, e.g. by applying vacuum, by evaporating in the presence of desiccating agents, such as dry silica, calcium chloride, "diphosphor pentoxide" or molecular sieve or combinations of two or more of these, or by passing through of dry gases, such as nitrogen, and/or lyophilization. Preferably, the concentration takes place such that the corresponding extracts or extract combinations are obtained as concentrated solutions with a specific gravity of 1.15 to 1.35, e.g. 1.2 to 1.3 (relative gravity preferably given in g/ml).

Where the component is a powder, the powder is either commercially obtained or obtained by grinding of the plants or plant parts, e.g. roots or the like, that serve as starting material for the component in powder form, alone or together with one or more different plants or plant parts that serve for one or more of the other components used in the manufacture of the products or formulations according to the invention.

The process allowing to produce the products of the invention is preferably conducted so that the extracts of the components mentioned above are mixed, as pre-formed mixtures (e.g. from extraction as described above of one or more starting materials in the same batch) and/or as single components, with the powder components which may also be used as single components or as mixtures of one or more of the components, and with a pharmaceutically acceptable carrier material. A further step may be the formation of unit dosage forms, such as capsules or especially tablets (pills), which coating step with a coating that may be coloured in order to be able to distinguish dosage forms that for the whole or part of a formulation of product of the invention. In the case of a product of the invention with more than one unit dosage form, the manufacture may further comprise the packaging of the one or more components and/or the preparation of accompanying information (e.g. in the form of package leaflets) that recommend or describe the preferred use of the dosage forms simultaneously, sequentially or (in a less preferred mode of the invention) separately.

Preferably, the process is as described in Examples 1, 2 or 3, but using the ranges of relative part per weight amounts are as described above in paragraphs (F), (I) or (L), respectively, and allowing also different colours of the resulting tablets.

### Examples:

The following examples illustrate the invention.

### Example 1: "White Tablets" for use in AIDS treatment

The tablet is produced as follows from the following ingredients in the following amounts (formula for pill basis):

| | |
|---|---|
| (1) Isatis root (from Isatis tinctoria, Chinese: Ban Lan Gen) | 36 g |
| (2) Glycyrrhizae radix (licorice; from Glycyrrizae uralensis, Chinese: Gan Cao) | 51 g |
| (3) Trichosanthis Radix (powder form), | |
| Chinese: Tian huafeng (root from Trichosanthis kirilowii Maxim) | 24 g |
| (4) Trichosanthis Kirilowii Maxim fruit (Chinese: Gualou) | 24 g |
| (5) Rhizoma pinelliae (rhizome of Pinellia ternata, Chinese Ban Xia) | 3 g |
| (6) Zingiberis Siccatum rhizoma (Ginger Root in dried form, Chinese: Gan Jiang) | 3 g |

Top quality herbs are selected according to the formula for the pill basis given above.

Ingredients (1), (2), (4), (5) and (6) (the latter two serving also against nausea caused e.g. by component (4)) mentioned above are transferred into a vacant extract container. Using steam, they are boiled for 2 hours after mixing for 0.5 hours with water (herb:water = 1:10 by weight). The resulting mixture is passed through a filter (pore size 3 x 3 mm), and the liquid is separated from the boiled raw materials and is put aside (first extract batch). Then a further amount of water is added (herbs:water = 1:8 by weight). Using steam, the material is boiled again for 2h. The filtering procedure is repeated and the liquid extract (second extract batch) is obtained from the extract container.

Then the first and second extract batch of boiled liquid thus obtained are collected in a deposit container after removal of the dust deposit formed on the first boiled liquid. The liquid mixture of the two extract batches is kept overnight in the deposit container.

After this, the clear water on the top level of the liquid extract is removed carefully. The remaining liquid is put into a concentrating container to concentrate the liquid form of the extract to obtain a concentrated solution with a specific gravity of 1.2 to 1.3, resulting in a concentrated solution.

After that, ingredient (3) is ground to form a powder and then added to the concentrated solution form extract of specific gravity 1.2 to 1.3.

A part (5 weight-%) of white fine wheat starch powder for tablet formation (tablet forming powder used for forming the tablets and in order to allow dissolution and rupturing in the stomach) is added to the material in a drying container for forming the tablets.

The dried plant-derived mixture from the drying container is then ground in order to mix extract powder and added tablet forming powder. After thorough mixing, the resulting mass (herbally derived mixture) is formed into small pellets and is then used for forming tablets by pressing the mass in a standard tablet forming device so that each tablet contains 1000 mg of the resulting mass.

Finally, the tablets are coated using sugar in combination with a white sugar pigment and talc powder (sugar:talc powder = 1:19), the weight relation between herbally derived mixture and coating being 75:25 or less.

The final product (White Tablet) is packaged. It should be kept dry and at room temperature until use. It is stable for up to five years.

The dosage to be applied to an adult patient is for example 4000 mg (4 tablets) three times a day after meals, the dosage for children half the dose for adult persons. The treatment can be without further formulations or in parallel to treatment with the tablets of Examples 2 and/or 3. The term or duration of treatment is, for example, from 3 to 12 months, preferably more than 6 months.

### Example 2: "Red Tablets" for use in the treatment of AIDS:

The tablet is produced as follows from the following ingredients:

| | |
|---|---|
| (A) Panacis quinquefolia dry powder (American Ginseng, Panax quinquefolius) | 20 g |
| (B) Angelicae sinensis (Olive.) Diels.(powder form the root, Chinese Deng gui) | 48 g |
| (C) Cordyceps sinensis (Berk.) (powdered form of Cordyceps sinensis, Aweto; Chinese Dong Chong Xia Cao) ) | 0.5 g |
| (D) Astragali Radix (Astragalus membranaceu (Fisch.) Bunge Chinese: Huang Qi) | 45 g |
| (E) Salviae miltiorrhizae Radix (from Salvia miltiorrhiza, Chinese: Dan Shen) | 18 g |
| (F) Glycyrrhizae baked with honey (Licorice = Glycyrrhiza uralensis, baked with honey; Chinese Zhi Gan Cao) | 15 g |

Using the same procedure as described in Example 1 for ingredients (1), (2), (4), (5) and (6), first from ingredients (D), (E) and (F) an extract in the form of a concentrated solution of specific gravity 1.2 to 1.3 is formed. On the other hand, ingredients (A), (B) and (C) are first ground into powder form and then added to the dried extract. Tablet formation is as in Example 1 (addition of white tablet forming powder, pressing of the tablets and coating), however, a red pigment sugar is added for colouring the coating red.

Packing and storage are as described for White Tablets in Example 1.

The recommended dosages for adults are 4000 mg (4 tablets) given three times a day after meals for adult persons, half this dosage for children. The treatment can be without further formulations or in parallel to treatment with the tablets of Examples 1 and/or 3. Treatment duration is preferably as in Example 1.

### Example 3: Combination Tablets (Yellow Tablet):

Using the ingredients (1), (2), (4), (5) and (6) in the amounts given in Example 1 and the ingredients (D), (E) and (F) in the amounts given in Example 2, first an extract is formed of specific gravity 1.2 to 1.3 as described in Examples 1 and 2. Then, the ground components (3) from Example 1 and (A), (B) and (C) from Example 2 are added. From the resulting mixture, after addition of white tablet forming powder tablets containing 1000 mg each of the mixture are pressed as described in Example 1. Finally, a yellow coating is added.

The packing and storage is as described in Example 1.

The recommended dosage is 8 yellow tablets thrice daily after meals for adults, for children half this dosage. Treatment duration is preferably as in Example 1.

### Example 4: Treatment of AIDS Patients with Red and White Tablets

Clinically, White Tablets (Example 1) and Red Tablets (Example 2) are administered to patients in combination. Parallel data for treatment with only one of these tablet types each is also possible (data not shown).

Where indicated, Viral Load in copies per ml has been measured using the Quantitative HIV PCR - Baseline Roche Amplicor® Test Vers. 1.5 (Roche Diagnostics, Switzerland), a quantitative HIV test based on Polymerase Chain Reaction detecting virus genomes (RNA). Results are given in RNA copies/ml. CD4 counts are measured by dying and counting according to standard procedures.

Among a number of other patients with correspondingly good treatment results, for which the results are not reported here, the following patients have been treated (based on compassionate use with patient information and consent):

### Patients #1 and #2:

The patients are a couple from Zambia. #1 is a male born in 1966, #2 a female born in 1973. Both have their blood tested before and during therapy with Red Tablets and White Tablets according to Examples 1 and 2. Treatment started on April 16, 2003. To each of them five White Tablets and five Red Tablets have been and are being administered three times daily after meals. The patients are still on therapy.

Results of Viral Load/CD4 counting:

| Patient and time point | Viral Load (RNA copies/ml) | CD4 Count (Helper) (cells/µl blood) |
|---|---|---|
| #1 May 2, 2003 | 198 000 | 95 |
| # 1 Nov. 3, 2003 | 112 000 | 150 |
| # 1 Aug. 2, 2004 | 26 500 | 163 |
| #2 May 2, 2003 | 17 000 | 94 |
| # 2 Nov 3, 2003 | 4 950 | 180 |
| # 2 Aug. 2, 2004 | 10 800 | 267 |

The normal value for CD4 counts in the test system used for healthy persons is 600 to 1500 cells/µl blood.

In case of both patients #1 and #2, a strong reduction of the Viral Load is found, accompanied by a strong increase in CD4 count. This shows that the treatment with the combination of White and Red Tablets shows a strong effect against AIDS in both cases.

### Patient #3:

The patient is from Windhoik, Namibia, and has been receiving four White Tablets and four Red Tablets according to Examples 1 and 2 three times daily from Dec. 6, 2001 to March 6, 2002, and from May 8, 2003, to November 8, 2003. He restarted therapy on April 24, 2004 and is presently still under treatment.

The patient reports that his health has improved significantly. He does no longer have frequent attacks of painful sores and lesions on his legs, eyes, eye balls, inside his ears and around his genitals, or persistent skin rashes, all of which he showed before treatment. He only gets an occasional sore or pimple here and there, but these are not much painful and heal off quickly, other than in the past where they lasted for long periods and caused much pain. He no longer sweats profusely during the night, whereas before treatment he usually woke up on a wet pillow and wet bed sheets. His body weight increased steadily from 70 kg to now around 90± 3 kg (depending on whether he travels a lot and thus has less occasion to eat good meals or not). He no longer feels tired out and does no longer experience body pains as was the case before he took the capsules. His appetite has increased significantly, whereas in the past it was very poor. His skin looks fresh and healthy, unlike in the past when it was "dusty" and always full of rashes and sores, especially in the region where a beard normally grows.

The report of the symptoms together with the comparison to the original symptoms suggest that, in spite of the treatment interruptions, the treatment still is effective and shows no signs of resistance development.

### Patient # 4:

This patient is at treatment start is a male from Holland born in 1973. He started anti-HIV therapy in May 2002, taking 4 White Tablets and 4 Red Tablets three times daily after meals. When the patient was introduced to the doctor for the first time, he was in very bad condition. He showed the symptoms of almost full blown AIDS stage at that time. Physically, he displayed a weight loss of more than 10 kg, weakness, poor appetite and cough, sweating a lot. Laboratory tests results were: CD4 absolute count less than 350 cells/µl, Viral Load more than 50 000 copies/ml.

After 23 months therapy with White and Red Tablets that at the time of filing of this application is still ongoing, he has gained almost 10 kg of weight (weighing more than 70 kg now). All of the previous physical symptoms have disappeared. He can work full time and goes to the gymn three times a week. He is happy and enjoys his regained normal life. Blood tests in January 2004 show that the CD4 absolute count is more than 900 cells/µl and the Viral Load has strongly decreased to 70 copies/ml.

Thus there is a strong improvement, the CD4 counts especially having risen above the critical threshold of around 350 cells/µl, while the Viral Load has decreased to very low values.

### Example 5: In vitro assay to confirm hindrance of cell fusion by Red or White or Red and White Tablet:

### Principle of the assay:

HIV enters the cell by fusion of the virus lipid membrane with the outer membrane of the host cell. The fusion is brought about by interaction of 4 membrane exposed proteins: the two virus surface glycoproteins gp120 and gp41, and the cellular attachment receptor CD4 and the cellular fusion receptors CXCR4 and CCR5).

To visualize such a fusion event in the microscope, cells have been construed which express both viral proteins continuously. If these cells are cultured together with HIV-infectable cells that express the receptors, the cells fuse spontaneously. As a result, giant cells (syncytia) with multiple nuclei are formed which can be counted in the microscope after staining.

### Materials:

HeLa-SX22-1 cells/CD-4-CCR5+CXR4 (see Koch, E.C., et al., Antiviral Therapy 8, 485-487 (2003); Walker et al., Antiviral Therapy 8, 463-470 (2003); and Klimkait et al., Arch. Virol. 143, 2109-2131 (1998)). These cells contain a reporter gene with a beta-Galactosidase gene downstream of the HIV-LTR region that is under regulation by HIV TAT, so that when the cells are infected they can be stained blue by addition of X-Gal (5-bromo-4-chloro-3-indolyl-β-D-galactopyranoside).

CL-4/gp120+gp41 cells: These cells are well-known as cells expressing the HIV envelope protein and thus a virus-free HIV model system, see Kräusslich, H.G., et al., Virology 192, 605-617 (1993).

### Culturing:

As controls, HeLa cells are grown in standard Dulbecco's Minimal Essential Medium (DMEM) without selection.

The HeLa-SX22-1 cells are grown in standard DMEM with 1 µg puromycine, 1 mg/ml antibiotic geneticin (G418).

The CL-4 cells are grown in standard DMEME without selection.

### Assay procedure:

Both cell types (HeLa or HeLa-SX22-1 and CL-4) are trypsinized ans suspended in 3 ml medium and counted. A suspension with 1 million cells each in DMEM is made.

The trypsinized HeLa/HeLa-SX22-1 cells are washed once in 10 ml DMEM without G418 and puromycin.

The HeLa or HeLa-SX22-1 cells are diluted in standard DMEM to 100 000 cells/ml.

CL-4 cells are diluted 3-fold.

The CL-4 cells (12 ml) and the HeLa or the HeLa-SX22-1 cells (6 ml) are mixed with each other at a ration of 2:1.

150 µl of the resultant cell mix is added to all wells of a 48-well microtiter plate (about 15 000 cells/well). 450 µl standard DMEM medium are added to each well.

Humic acid analogue HS-1500 (see Schneider, J., et al., Virology 218, 389-295 (1996) and EP 0 537 430 A) is weighed in at 2.2 mg/ml in phosphate buffered saline (PBS), followed by 10-fold dilution with standard DMEM and then by two-fold serial dilution: 220; 110; 55; 27.5; 13.75 µg/ml, from this 200 µl are added to 600 µl of cell culture resulting in final concentrations of 27.5; 13.7; 6.9; 3.4; 1.7 µg/ml.

Red or White or Red and White Tablet Extracts are prepared as follows (Red and White Tablets are those described in Examples 1 and 2) :

A stock solution containing 81 mg/ml extracted from either Red Tablets, White Tablets or the combination of Red and White Tablets is prepared by grinding the pills in with a pestle in a porcellain Mortar at room temperature for 3 to 4 min. Phosphate buffered saline is added and the grinding continued for 1 min. The resulting slurry is sedimented in a centrifuge at 3 500 x g for 5 min. The supernatant is filtered through a 0.22 µm pore size membrane. The filtrate is kept at 4 ° C until use (up to 1 day later). The mentioned concentration is determined by lyophilization and weighing of the tubes. For each of these mother solutions, a 3-fold dilution is made in standard DMEM to a concentration of 27 mg/ml. From these solutions, a 2-fold dilution series is made to obtain, in addition to the 27 mg/ml stock solution, 13.6; 6.8; 3.4; and 1.7 mg/ml-solutions.

200 µl of these stock solutions are added in duplicate to 600 µl of culture per well, respectively. The final concentration of the tablet extracts in the culture is thus 6.75; 3.4; 1.7; 0.84; 0.42 mg/ml.

The cells are cultured for 20 h, fixed and stained with Hemacolor® (a reagent for staining blood smears; Merck, Darmstadt, FRG). The cells are washed once with PBS, then kept at 4 °C until counting. Syncytia with more than 4 cell nuclei are counted.

At the highest concentration, the tablet extracts are toxic, thus no syncytia counting is made and only results with 4 concentrations (3.4 = A; 1.7 = B; 0.84 = C; and 0.42 mg/ml =D in the table under results) are obtained.

### Results:

The number of syncytia counted for each inhibitor concentration are given in the following table (with inhibitors, mean of duplicates):

| Inhibitor concentration | Syncytia with Red Tablet Extract | Syncytia with White Tablet Extract | Syncytia with mixed White + Red Tablet Extract | Without inhibitor (mean value of 7 wells without inhibitor) |
|---|---|---|---|---|
| A | 14.5 | 7.5 | 11 | |
| B | 35 | 33 | 42 | |
| C | 68 | 42.5 | 51 | |
| D | 62 | 72.5 | 74 | |
| | | | | 93.3 |

For comparison, in the presence of HS-1500 the following mean values of duplicate determinations of syncytia counts are found:

For 13.7 µl/mg: 0; for 6.9 µg/ml: 9; for 3.4 µg/ml: 49; for; 1.7 µg/ml: 94.

The EC₅₀ concentrations (concentration of half-maximum inhibition of syncytium formation compared to controls with HeLa cells) are:

| Active Agent | EC₅₀ value |
|---|---|
| White Tablet Extract | 0.86 (mg/ml) |
| Red Tablet Extract | 1.58 (mg/ml) |
| White/Red Tablet mix | 1.50 (mg/ml) |
| HS-1500 | 6.9 µg/ml |

Differences by a factor of 2 are not necessarily significant. The humic acid compound HS-1500 is more effective, but also more toxic. In this assay, the EC₅₀ values are higher than in an infection assay with active virus (data not shown), for White Tablet extract about 2 times, for Red Tablets about 10 times higher. Such ratios have been observed with other inhibitors of the humic acid class as well. The reason may be that interaction of cells involves more receptor-ligand pairs than interaction of a (small) virus with a cell.

Discussion: The tablet extracts block HIV-induced fusion. Therefore, also fusion between virus and cells is inhibited. Thus Fusion Inhibition is at least one potential mechanism by which the tablets inhibit HIV. Inhibition at the first step of infection (virus entry) may be an advantage over inhibitors that interfere only with later stages of the viral replication cycle.

## Claims

1. A pharmaceutical product containing
(i) either one single dosage form,
or
(ii) two dosage forms jointly active against the disease to be treated as kit of parts for simultaneous, and/or sequential use, where each dosage form comprises 4 to 8 of the following components, so that each of
the single dosage form (i) alone,
or the two dosage forms as kit of parts (ii) when taken together,
contains all of the following components in the following relative part per weight amounts:
an extract of Isatis root 10 - 60,
an extract of Glycyrrhizae radix 20 - 80,
an extract of Trichosanthis Kirilowii fruit 5 - 45,
an extract of Rhizoma pinelliae 0.5 - 10,
an extract of Zingiberis Siccatum rhizome 1 - 6,
an extract of Astragali radix 10 - 80,
an extract of Salvia miltiorrhizae radix 3 - 35,
an extract of Glycyrrhizae baked with honey 2 - 40,
Trichosanthis Kirilowii radix in powder form 5 - 45,
Panax quinquefolia in powder form 3 - 50,
Angelica sinensis in powder form 20 - 80 and
Cordyceps sinensis in powder form 0.05 - 5;
and optionally one or more pharmaceutically acceptable
carrier and/or coating materials, with or without further additives in accordance with accepted practices of pharmaceutical formulating,
for use in the treatment of a retroviral infection.

2. The product according to claim 1 as kit of parts with two dosage forms for sequential or preferably simultaneous administration.

3. The product according to any one of claims 1 or 2 wherein each dosage form is a capsule or preferably a tablet.

4. The product according to any one of claims 1 or 2 wherein the extract components are prepared from a concentrated solution with a specific gravity of 1.15 to 1.35, preferably 1.2 to 1.3, before the final drying.

5. The product according to any one of claims 1 to 4 wherein the extract components are obtainable by water extraction.

6. The product according to any one of claims 1 to 5 wherein the components are present in the following relative parts per weight amounts:
extract of Isatis root 20 to 50, preferably 30 to 40, most preferably 36;
extract of Glycyrrhizae radix 30 to 70, preferably 45 to 55, most preferably 51;
extract of Trichosanthis Kirilowii fruit 15 to 35, preferably 20 to 30, most preferably 24;
extract of Rhizoma pinelliae 1 to 6, preferably 1.5 to 5, most preferably 3;
extract of Zingiberis Siccatum rhizoma in dried form, preferably 1.5 to 5, most preferably 3;
extract of Astragali radix 20 to 70, preferably 35 to 55, most preferably 45;
extract of Salvia miltiorrhizae radix 5 to 30, preferably 10 to 25, most preferably 18;
extract of Glycyrrhizae baked with honey 5 to 30, preferably 10 to 20, most preferably 15;
Trichosanthis Kirilowii radix in powder form 15 to 35, preferably 20 to 30, most preferably 24;
Panax quinquefolia in powder form 5 to 35, preferably 10 to 30, most preferably 20;
Angelica sinensis in powder form 30 to 70, preferably 40 to 60, most preferably 48; and
Cordyceps sinensis in powder form 0.1 to 1, preferably 0.25 to 0.75, most preferably 0.5.

7. A product in the form of a kit of parts according to claim 1, wherein the two dosage forms are:
a) a pharmaceutical formulation, especially a tablet or capsule, comprising the following components in the following relative part per weight amounts:
an extract of Isatis root 10 to 60,
an extract of Glycyrrhizae radix 20 to 80,
an extract of Trichosanthis Kirilowii fruit 5 to 45,
an extract of Rhizoma pinelliae 0.5 to 10,
an extract of Zingiberis Siccatum rhizoma in dried form 1 to 6, and
Trichosanthis Kirilowii radix in powder form 5 to 45,
and optionally one or more pharmaceutically acceptable carrier and/or coating materials; and
b) a pharmaceutical formulation, especially a tablet or capsule, comprising the following components in the following relative part per weight amounts:
an extract of Astragali radix 10 to 80,
an extract of Salvia miltiorrhizae radix 3 to 35,
an extract of Glycyrrhizae baked with honey 2 to 40,
Panax quinquefolia in powder form 3 to 50,
Angelica sinensis in powder form 20 to 80 and
Cordyceps sinensis in powder form 0.05 to 5,
and optionally one or more pharmaceutically acceptable carrier and/or coating materials.

8. A product in the form of a kit of parts according to claim 7, wherein the pharmaceutical formulations of a) and b) each are in the form of a tablet or capsule wherein the extract components are prepared from a concentrated solution with a specific gravity of 1.15 to 1.35, preferably 1.2 to 1.3, before the final drying.

9. A product in the form of a kit of parts according to claim 7 or 8, wherein the pharmaceutical formulations of a) and b) each are in the form of a tablet or capsule wherein the extract components are obtainable by water extraction.

10. A product in the form of a kit of parts according to any one of claims 7 to 9, wherein the pharmaceutical formulations of a) and b) each are in the form of a tablet or capsule wherein the components are present in the following relative parts per weight amounts:
in component a) :
extract of Isatis root 20 to 50, preferably 30 to 40, most preferably 36;
extract of Glycyrrhizae radix 30 to 70, preferably 45 to 55, most preferably 51;
extract of Trichosanthis Kirilowii fruit 15 to 35, preferably 20 to 30, most preferably 24;
extract of Rhizoma pinelliae 1 to 6, preferably 1.5 to 5, most preferably 3;
extract of Zingiberis Siccatum rhizome in dried form 1.5 to 5, most preferably 3; and
Trichosanthis Kirilowii radix in powder form 15 to 35, preferably 20 to 30, most preferably 24; and
in component b):
extract of Astragali radix 20 to 70, preferably 35 to 55, most preferably 45;
extract of Salvia miltiorrhizae radix 5 to 30, preferably 10 to 25, most preferably 18;
extract of Glycyrrhizae baked with honey 5 to 30, preferably 10 to 20, most preferably 15;
Panax quinquefolia in powder form 5 to 35, preferably 10 to 30, most preferably 20;
Angelica sinensis in powder form 30 to 70, preferably 40 to 60, most preferably 48; and Cordyceps sinensis in powder form 0.1 to 1, preferably 0.25 to 0.75, most preferably 0.5.

11. A product according to claim 7 comprising as dosage form a) a tablet or capsule and as dosage form b) a tablet or capsule for the simultaneous or sequential treatment of HIV infection of a warm-blooded animal, especially a human.

12. A product according to any one of claims 1 to 11 for use in the treatment of AIDS.

## Patentansprüche

1. Pharmazeutisches Produkt, das folgendes enthält:
(i) entweder eine Einzeldosisform
oder
(ii) zwei Dosisformen, die gemeinsam gegen die zu behandelnde Krankheit als aus Teilen bestehendes Kit für die gleichzeitige bzw. aufeinanderfolgende Verwendung wirksam sind, wobei jede Dosisform 4 bis 8 der folgenden Bestandteile umfaßt, so daß jeweils
die Einzeldosis (i) allein
oder die zwei Dosisformen als aus Teilen bestehendes Kit (ii) bei gemeinsamer Einnahme
alle der folgenden Bestandteile in den folgenden relativen Gewichtsteilen enthalten:
Extrakt aus Isatis-Wurzel 10 - 60,
Extrakt aus Glycyrrhizae Radix 20 - 80,
Extrakt aus Trichosanthis-kirilowii-Frucht 5 - 45,
Extrakt aus Rhizoma Pinelliae 0,5 - 10,
Extrakt aus Zingiberis-siccatum-Rhizom 1 - 6,
Extrakt aus Astragali Radix 10 - 80,
Extrakt aus Salviae-miltiorrhizae-Radix 3 - 35,
Extrakt aus Glycyrrhiza, mit Honig gegart 2 - 40,
Trichosanthis-kirilowii-Radix in Pulverform 5 - 45,
Panax quinquefolia in Pulverform 3 - 50,
Angelica sinensis in Pulverform 20 - 80 und
Cordyceps sinensis in Pulverform 0,05 - 5;
sowie gegebenenfalls einen oder mehrere pharmazeutisch annehmbare Träger und/oder Beschichtungsmaterialien, mit oder ohne weitere galenisch annehmbare Zusatzstoffe,
für die Verwendung bei der Behandlung einer Retrovirusinfektion.

2. Produkt nach Anspruch 1 als aus Teilen bestehendes Kit mit zwei Dosisformen für die aufeinanderfolgende oder vorzugsweise gleichzeitige Verabreichung.

3. Produkt nach einem der Ansprüche 1 oder 2, wobei es sich bei jeder Dosisform um eine Kapsel oder vorzugsweise eine Tablette handelt.

4. Produkt nach einem der Ansprüche 1 oder 2, wobei die Extraktbestandteile aus einer konzentrierten Lösung mit einer spezifischen Dichte von 1,15 bis 1,35, vorzugsweise 1,2 bis 1,3, vor der abschließenden Trocknung hergestellt werden.

5. Produkt nach einem der Ansprüche 1 bis 4, wobei die Extraktbestandteile durch Extraktion mit Wasser erhältlich sind.

6. Produkt nach einem der Ansprüche 1 bis 5, wobei die Bestandteile in den folgenden relativen Gewichtsteilen vorliegen:
Extrakt aus Isatis-Wurzel 20 bis 50, vorzugsweise 30 bis 40, am stärksten bevorzugt 36;
Extrakt aus Glycyrrhizae Radix 30 bis 70, vorzugsweise 45 bis 55, am stärksten bevorzugt 51;
Extrakt aus Trichosanthis-kirilowii-Frucht 15 bis 35, vorzugsweise 20 bis 30, am stärksten bevorzugt 24;
Extrakt aus Rhizoma Pinelliae 1 bis 6, vorzugsweise 1,5 bis 5, am stärksten bevorzugt 3;
Extrakt aus Zingiberis-siccatum-Rhizom in getrockneter Form, vorzugsweise 1,5 bis 5, am stärksten bevorzugt 3;
Extrakt aus Astragali Radix 20 bis 70, vorzugsweise 35 bis 55, am stärksten bevorzugt 45;
Extrakt aus Salviae-miltiorrhizae-Radix 5 bis 30, vorzugsweise 10 bis 25, am stärksten bevorzugt 18;
Extrakt aus Glycyrrhiza, gegart mit Honig, 5 bis 30, vorzugsweise 10 bis 20, am stärksten bevorzugt 15;
Trichosanthis-kirilowii-Radix in Pulverform 15 bis 35, vorzugsweise 20 bis 30, am stärksten bevorzugt 24;
Panax quinquefolia in Pulverform 5 bis 35, vorzugsweise 10 bis 30, am stärksten bevorzugt 20;
Angelica sinensis in Pulverform 30 bis 70, vorzugsweise 40 bis 60, am stärksten bevorzugt 48; und
Cordyceps sinensis in Pulverform 0,1 bis 1, vorzugsweise 0,25 bis 0,75, am stärksten bevorzugt 0,5.

7. Produkt in der Form eines aus Teilen bestehenden Kits nach Anspruch 1, wobei es sich bei den zwei Dosisformen um folgendes handelt:
a) eine Darreichungsform, insbesondere eine Tablette oder Kapsel, umfassend die folgenden Bestandteile in den folgenden relativen Gewichtsteilen:
Extrakt aus Isatis-Wurzel 10 bis 60,
Extrakt aus Glycyrrhizae Radix 20 bis 80,
Extrakt aus Trichosanthis-kirilowii-Frucht 5 bis 45, Extrakt aus Rhizoma Pinelliae 0,5 bis 10,
Extrakt aus Zingiberis-siccatum-Rhizom in getrockneter Form 1 bis 6 und
Trichosanthis-kirilowii-Radix in Pulverform 5 bis 45
sowie gegebenenfalls einen oder mehrere pharmazeutisch annehmbare Träger und/oder Beschichtungsmaterialien; und
b) eine Darreichungsform, insbesondere eine Tablette oder Kapsel, umfassend die folgenden Bestandteile in den folgenden relativen Gewichtsteilen:
Extrakt aus Astragali Radix 10 bis 80,
Extrakt aus Salviae-miltiorrhizae-Radix 3 bis 35,
Extrakt aus Glycyrrhiza, mit Honig gegart 2 bis 40,
Panax quinquefolia in Pulverform 3 bis 50,
Angelica sinensis in Pulverform 20 bis 80 und
Cordyceps sinensis in Pulverform 0,05 bis 5
sowie gegebenenfalls einen oder mehrere pharmazeutisch annehmbare Träger und/oder Beschichtungsmaterialien.

8. Produkt in Form eines aus Teilen bestehenden Kits nach Anspruch 7, wobei die Darreichungsform von a) und b) jeweils in Form einer Tablette oder Kapsel vorliegt, wobei die Extraktbestandteile aus einer konzentrierten Lösung mit einer spezifischen Dichte von 1,15 bis 1,35, vorzugsweise 1,2 bis 1,3, vor dem abschließenden Trocknen hergestellt werden.

9. Produkt in Form eines aus Teilen bestehenden Kits nach Anspruch 7 oder 8, wobei die Darreichungsformen von a) und b) jeweils in Form einer Tablette oder Kapsel vorliegen, wobei die Extraktbestandteile durch Extraktion mit Wasser erhältlich sind.

10. Produkt in Form eines aus Teilen bestehenden Kits nach Anspruch 7 oder 9, wobei die Darreichungsformen von a) und b) jeweils in Form einer Tablette oder Kapsel vorliegen, wobei die Bestandteile in den folgenden relativen Gewichtsteilen vorliegen:
in Bestandteil a):
Extrakt aus Isatis-Wurzel 20 bis 50, vorzugsweise 30 bis 40, am stärksten bevorzugt 36;
Extrakt aus Glycyrrhizae Radix 30 bis 70, vorzugsweise 45 bis 55, am stärksten bevorzugt 51;
Extrakt aus Trichosanthis-kirilowii-Frucht 15 bis 35, vorzugsweise 20 bis 30, am stärksten bevorzugt 24;
Extrakt aus Rhizoma Pinelliae 1 bis 6, vorzugsweise 1,5 bis 5, am stärksten bevorzugt 3;
Extrakt aus Zingiberis-siccatum-Rhizom in getrockneter Form, vorzugsweise 1,5 bis 5, am stärksten bevorzugt 3; und
Trichosanthis-kirilowii-Radix in Pulverform 15 bis 35, vorzugsweise 20 bis 30, am stärksten bevorzugt 24; und
in Bestandteil b):
Extrakt aus Astragali Radix 20 bis 70, vorzugsweise 35 bis 55, am stärksten bevorzugt 45;
Extrakt aus Salviae-miltiorrhizae-Radix 5 bis 30, vorzugsweise 10 bis 25, am stärksten bevorzugt 18;
Extrakt aus Glycyrrhiza, gegart mit Honig, 5 bis 30, vorzugsweise 10 bis 20, am stärksten bevorzugt 15;
Panax quinquefolia in Pulverform 5 bis 35, vorzugsweise 10 bis 30, am stärksten bevorzugt 20;
Angelica sinensis in Pulverform 30 bis 70, vorzugsweise 40 bis 60, am stärksten bevorzugt 48; und Cordyceps sinensis in Pulverform 0,1 bis 1, vorzugsweise 0,25 bis 0,75, am stärksten bevorzugt 0,5.

11. Produkt nach Anspruch 7, umfassend als Dosisform a) eine Tablette oder Kapsel und als Dosisform b) eine Tablette oder Kapsel für die gleichzeitige oder aufeinanderfolgende Behandlung von HIV-Infektion eines Warmblüters, insbesondere eines Menschen.

12. Produkt nach einem der Ansprüche 1 bis 11 für die Verwendung bei der Behandlung von AIDS.

## Revendications

1. Produit pharmaceutique contenant
(i) soit une forme de dosage unique,
soit
(ii) deux formes de dosage conjointement actives contre la maladie à traiter sous forme de kit de parties pour une utilisation simultanée et/ou séquentielle, chaque forme de dosage comprenant quatre à huit des composants suivants, de sorte que chacun parmi
la forme de dosage unique (i) seule,
ou les deux formes de dosage sous forme de kit de parties (ii) lorsqu'elles sont prises ensemble,
contient tous les composants suivants dans les quantités en parties en poids relatives suivantes :
un extrait de racine d'Isatis 10 - 60,
un extrait de Glycyrrhiza radix 20 - 80,
un extrait de fruit de Trichosanthis kirilowii 5 - 45,
un extrait de Rhizoma pinelliae 0,5 - 10,
un extrait de rhizome de Zingiberis Siccatum 1 - 6,
un extrait d'Astragali radix 10 - 80,
un extrait de Salviae miltiorrhizae radix 3 - 35,
un extrait de Glycyrrhiza cuit au four avec du miel 2 - 40,
Trichosanthis kirilowii radix en poudre 5 - 45,
Panax quinquefolia en poudre 3 - 50,
Angelica sinensis en poudre 20 - 80, et
Cordyceps sinensis en poudre 0,05 - 5,
et éventuellement un ou plusieurs matériaux de support et/ou d'enrobage pharmaceutiquement acceptables, avec ou sans d'autres additifs conformément aux pratiques acceptées de la formulation pharmaceutique,
pour une utilisation dans le traitement d'une infection rétrovirale.

2. Produit selon la revendication 1 sous forme de kit de parties avec deux formes de dosage pour l'administration séquentielle ou de préférence simultanée.

3. Produit selon l'une ou l'autre des revendications 1 et 2, **caractérisé en ce que** chaque forme de dosage est une capsule ou de préférence un comprimé.

4. Produit selon l'une ou l'autre des revendications 1 et 2, **caractérisé en ce que** les composants des extraits sont préparés à partir d'une solution concentrée ayant un poids spécifique de 1,15 à 1,35, de préférence 1,2 à 1,3, avant le séchage final.

5. Produit selon l'une quelconque des revendications 1 à 4, **caractérisé en ce que** les composants des extraits peuvent être obtenus par extraction à l'eau.

6. Produit selon l'une quelconque des revendications 1 à 5, **caractérisé en ce que** les composants sont présents dans les quantités en parties en poids relatives suivantes :
extrait de racine d'Isatis 20 à 50, de préférence 30 à 40, de façon tout à fait préférable 36 ;
extrait de Glycyrrhiza radix 30 à 70, de préférence 45 à 55, de façon tout à fait préférable 51 ;
extrait de fruit de Trichosanthis kirilowii 15 à 35, de préférence 20 à 30, de façon tout à fait préférable 24 ;
extrait de Rhizoma pinelliae 1 à 6, de préférence 1,5 à 5, de façon tout à fait préférable 3 ;
extrait de rhizome de Zingiberis Siccatum sous une forme séchée, de préférence 1,5 à 5, de façon tout à fait préférable 3 ;
extrait d'Astragali radix 20 à 70, de préférence 35 à 55, de façon tout à fait préférable 45 ;
extrait de Salviae miltiorrhizae radix 5 à 30, de préférence 10 à 25, de façon tout à fait préférable 18 ;
extrait de Glycyrrhiza cuit au four avec du miel 5 à 30, de préférence 10 à 20, de façon tout à fait préférable 15 ;
Trichosanthis kirilowii radix en poudre 15 à 35, de préférence 20 à 30, de façon tout à fait préférable 24 ;
Panax quinquefolia en poudre 5 à 35, de préférence 10 à 30, de façon tout à fait préférable 20 ;
Angelica sinensis en poudre 30 à 70, de préférence 40 à 60, de façon tout à fait préférable 48 ; et
Cordyceps sinensis en poudre 0,1 à 1, de préférence 0,25 à 0,75, de façon tout à fait préférable 0,5.

7. Produit sous forme de kit de parties selon la revendication 1, **caractérisé en ce que** les deux formes de dosage sont :
a) une formulation pharmaceutique, notamment un comprimé ou une capsule, comprenant les composants suivants dans les quantités en parties en poids relatives suivantes :
un extrait de racine d'Isatis 10 à 60,
un extrait de Glycyrrhiza radix 20 à 80,
un extrait de fruit de Trichosanthis kirilowii 5 à 45,
un extrait de Rhizoma pinelliae 0,5 à 10,
un extrait de rhizome de Zingiberis Siccatum sous une forme séchée 1 à 6, et
Trichosanthis kirilowii radix en poudre 5 à 45,
et éventuellement un ou plusieurs matériaux de support et/ou d'enrobage pharmaceutiquement acceptables ; et
b) une formulation pharmaceutique, notamment un comprimé ou une capsule, comprenant les composants suivants dans les quantités en parties en poids relatives suivantes :
un extrait d'Astragali radix 10 à 80,
un extrait de Salviae miltiorrhizae radix 3 à 35,
un extrait de Glycyrrhiza cuit au four avec du miel 2 à 40,
Panax quinquefolia en poudre 3 à 50,
Angelica sinensis en poudre 20 à 80 et
Cordyceps sinensis en poudre 0,05 à 5,
et éventuellement un ou plusieurs matériaux de support et/ou d'enrobage pharmaceutiquement acceptables.

8. Produit sous forme d'un kit de parties selon la revendication 7, **caractérisé en ce que** les formulations pharmaceutiques de a) et b) sont chacune sous forme d'un comprimé ou d'une capsule, les composants des extraits étant préparés à partir d'une solution concentrée ayant un poids spécifique de 1,15 à 1,35, de préférence 1,2 à 1,3, avant le séchage final.

9. Produit sous forme d'un kit de parties selon la revendication 7 ou 8, **caractérisé en ce que** les formulations pharmaceutiques de a) et b) sont chacune sous forme d'un comprimé ou d'une capsule, les composants des extraits pouvant être obtenus par extraction à l'eau.

10. Produit sous forme d'un kit de parties selon l'une quelconque des revendications 7 à 9, **caractérisé en ce que** les formulations pharmaceutiques a) et b) sont chacune sous forme d'un comprimé ou d'une capsule, les composants étant présents dans les quantités en parties en poids relatives suivantes :
dans le composant a)
extrait de racine d'Isatis 20 à 50, de préférence 30 à 40, de façon tout à fait préférable 36 ;
extrait de Glycyrrhiza radix 30 à 70, de préférence 45 à 55, de façon tout à fait préférable 51 ;
extrait de fruit de Trichosanthis kirilowii 15 à 35, de préférence 20 à 30, de façon tout à fait préférable 24 ;
extrait de Rhizoma pinelliae 1 à 6, de préférence 1,5 à 5, de façon tout à fait préférable 3 ;
extrait de rhizome de Zingiberis Siccatum sous une forme séchée, de préférence 1,5 à 5, de façon tout à fait préférable 3 ; et
Trichosanthis kirilowii radix en poudre 15 à 35, de préférence 20 à 30, de façon tout à fait préférable 24 ; et
dans le composant b)
extrait d'Astragali radix 20 à 70, de préférence 35 à 55, de façon tout à fait préférable 45 ;
extrait de Salviae miltiorrhizae radix 5 à 30, de préférence 10 à 25, de façon tout à fait préférable 18 ;
extrait de Glycyrrhiza cuit au four avec du miel 5 à 30, de préférence 10 à 20, de façon tout à fait préférable 15 ;
Panax quinquefolia en poudre 5 à 35, de préférence 10 à 30, de façon tout à fait préférable 20 ;
Angelica sinensis en poudre 30 à 70, de préférence 40 à 60, de façon tout à fait préférable 48 ; et
Cordyceps sinensis en poudre 0,1 à 1, de préférence 0,25 à 0,75, de façon tout à fait préférable 0,5.

11. Produit selon la revendication 7, comprenant à titre de forme de dosage a) un comprimé ou une capsule et à titre de forme de dosage b) un comprimé ou une capsule pour le traitement simultané ou séquentiel d'infection par le VIH chez un animal à sang chaud, notamment l'homme.

12. Produit selon l'une quelconque des revendications 1 à 12, destiné à être utilisé dans le traitement du SIDA.
